**Europäisches Patentamt**

**European Patent Office**

**Office européen des brevets**

(11) Publication number: **0 041 487**

**A1**

(12) # EUROPEAN PATENT APPLICATION

(21) Application number: **81850091.0**

(22) Date of filing: **25.05.81**

(51) Int. Cl.³: **A 61 M 25/00**
**A 61 M 1/00, A 61 F 5/44**

(30) Priority: **29.05.80 SE 8003985**
**23.12.80 SE 8009092**

(43) Date of publication of application:
**09.12.81 Bulletin 81/49**

(84) Designated Contracting States:
**AT BE CH DE FR GB IT LI LU NL SE**

(71) Applicant: **Scholander, Hans-Peter**
**Valhallavägen 12B**
**S-114 22 Stockholm(SE)**

(71) Applicant: **Scholander, Axel Fredrik**
**Mörbyleden 16**
**S-182 32 Danderyd(SE)**

(72) Inventor: **Scholander, Hans-Peter**
**Valhallavägen 12B**
**S-114 22 Stockholm(SE)**

(72) Inventor: **Scholander, Axel Fredrik**
**Mörbyleden 16**
**S-182 32 Danderyd(SE)**

(74) Representative: **Burman, Tore et al,**
**Bergling & Sundbergh AB P.O.Box 7645**
**S-103 94 Stockholm(SE)**

(54) Catheter device.

㊗ A catheter device (1) is disclosed which is intended either to work as a catheter per se or to work as a catheter adapter to be connected to a conventional catheter. The device is of that type which comprises a channel (5) intended for the liquid to be drained from a body cavity (2), and a channel for the inlet of gas, usually air, into the liquid channel (5) to break a liquid column in the liquid channel. An especially preferable use of the device according to the invention is for the drainage of a urinary bladder,

Previously known devices for the inlet of air have been provided with some form of a bacterium filter. According to the invention the need of a bacterium filter is completely eliminated. This is achieved by the fact that the gas channel (6) comprises two openings, the first one of which opens into a liquid collecting means (4) and the second one of which (9) opens into the liquid channel (5) upstream the outlet of the liquid channel into the liquid collecting means. Through the fact that the rest of the gas channel (6) is closed from the surrounding a totally closed gas circuit is obtained, wherein the gas intended for entrance into the liquid channel (5) is taken from the liquid collecting means (4). Thus, there will be no need of any gas from the outside.

According to a preferable embodiment of the device the gas channel (6) is dimensioned more narrow than the liquid channel (5).

*Fig.1*

TITLE:

## CATHETER DEVICE

TECHNICAL FIELD:

The present invention relates to a catheter device intended to be inserted into, or connected to an already existing catheter inserted in, a body cavity for drainage of fluid therefrom. In the present case the term catheter device, thus, means either a device, which functions as a catheter per se with improved function relative to previously known catheters, or a device which is intended to be adapted to a conventional catheter in order to improve the function thereof. In last-mentioned case a catheter attachment is in fact referred to.

The catheter device according to the invention is especially well suited for drainage of a urinary bladder, which is a body space or cavity, as it makes possible the drainage of the urinary bladder in a way which is physiologically more favourable than has hitherto been possible. The device is, however, not limited to this use but can advantageously be used also in other cases where the drainage of any animal body cavity is referred to. In this connection, it can also be added that nor is the catheter device . limited to the drainage of a liquid that is already present in the body cavity. It can of course, as is the case with conventional catheters, be used also to introduce a liquid into a body cavity, for instance for flushing the same, which liquid is then to be redrained therefrom. In that case the catheter device according to the invention can be utilized in a manner known per se for the introduction as well as the drainage of said liquid.

The device according to the invention is of that type which comprises a drainage means with at least one channel for liquid and at least one channel for inlet of gas into the liquid channel and a liquid storage or collecting means connected to the drainage means. The drainage means preferably comprises a tube or a pipe, said channels not necessarily having to be arranged in one and the same tube or pipe, but they can of course be comprised of separate tubes or pipes.

The gas which is introduced into the liquid channel is preferably air, as this represents a very simple embodiment, but of course the invention is applicable also to other gases. As the liquid collecting means there is preferably used a bag, for instance of a plastics material. As examples of other suitable liquid collecting means there can be mentioned plastics containers, glass bottles, etc.

BACKGROUND ART:

There are catheters of many different types and materials. Thus, rubber tubes have been used previously, but nowadays there are in common use different kinds of plastics materials, with or without surface treatments, for instance siliconization, inter alia to avoid incrustation of salts on the catheter surface, especially on the inside. For long time uses, catheters á demeure, CAD, catheters have been obtained in this way for insertion up to four months without any need of replacement.

The most unsofisticated type of catheter, the Nelaton catheter, simply consists of a single-channel tube, one end of which is introduced into the urinary bladder, and the other end of which is closed by a clip or a cock or is allowed to hang down into a container, for instance a urine bag.

In order to prevent the catheter from slipping or being pulled out of the urinary bladder after the insertion thereof, it can be equipped in the front portion thereof with an inflatable balloon, which is blown up through a separate channel in the catheter and acts as a stop towards retraction. The Foley catheter is an example of such a catheter type. Finally, the three--channel catheter can be mentioned, which in addition to the channels for urine drainage and inflation of the balloon possesses a third channel for the introduction of liquid and which is used for instance for flushing the urinary bladder, that is which permits a simultaneous liquid supply and drainage. If required, the catheter device according to the invention can include also these extra channels.

In a normally functioning urinary bladder a liquid pressure is built up in the bladder to a water column of about 100 millimeters, whereupon at a certain degree of filling of the bladder nerve impulses cause urinary contractions. Persons in health can control these contractions by controlling the external urinary bladder sphincter muscle which is will-controlled, so as to discharge the urine under control. With incontinent persons this is not the case, but urine is discharged unintentionally and uncontrolled. When using CAD the sphincter control is often replaced by a clip or a cock arranged in the catheter, which is opened at urinary contractions or at certain intervals. However, the requirement is that the patient itself is able to handle the opening or that nursing staff attends to the drainage at suitable times. When the bladder has been filled with such a large amount of urine that the patient feels contractions, or with atonic people is considered to be in need of a drainage of the bladder, the bladder can be drained by opening the clip or cock in the catheter. However, many elderly or retarded people cannot handle this by themselves, but personal assistance is required. However, since such assistance is not always available, the catheter is often allowed to

4

hang down into a urinary bag and the urine is allowed to drain continuously.

In this way, the bladder will not have any opportunity to be filled and thereby loses its normal ability to be filled with urine and thereafter be emptied. In view of this, the permanently empty bladder will become more and more shrivelled and finally it does not hold more than 50-100 milliliters. Through the fact that the drainage of the urine through the catheter takes place to a urinary bag located below the bladder via a continuous column of urine, there will be a vacuum in the bladder which deflates the bladder. This causes the catheter tip and the balloon to gall the wall of the bladder and give rise to inflammations, bleedings, pains, bladder cramps with leakage around the catheter and gives rise to scar formations. The end results will be a small thick-walled bladder which jams around the catheter and causes constant cramp and pain.

These inconveniences can be lessened if the negative pressure in the bladder is reduced, which can be achieved by entering gas, for instance air, into the urine channel of the catheter at a point downstreams the bladder, whereby a slight negative pressure will prevail in the bladder.

The letting in of the gas also enables a periodical filling of the bladder and an automatic drainage thereof, when the pressure in the bladder has reached a certain level. Hereby the nursing requirements will be reduced as to the patient and the personnel, and the risk of mismanagement will be lowered. Thus, normally the pressure in the bladder is approximately 100 millimeters as to column of water, and when contractions occur the pressure raises to 300-600 millimeters as to column of water. When using a common open catheter there is a constant negative pressure in the bladder, which causes the bladder to be contracted all the time. This in turn means that the bladder becomes shrivelled, as it is not allowed to work. By the inlet of gas the negative pressure is reduced, or is a pressure allowed to be built up to the desired level, before the drainage takes place. In last-mentioned case the bladder is refilled, and the drainage process is automatically repeated.

As an example of a device for the inlet of gas in connection with catheters intended for the drainage of urine, the gas preventing a column of urine to remain in the catheter, reference is made to the device that is disclosed in the Swedish patent application Serial No 7507047-4, which device relates to a catheter adapter having an aeration opening fitted with a filter. Other more or less sophisticated devices for the same or similar purposes are disclosed in the U.S. Patent specifications 3 419 009, 3 429 314, 3 598 124, 3 599 641, 3 604 420 and 3 690 315, which are mentioned in said Swedish patent application. Another device of this type, which has appeared on the market, is Cystomat $^{®}$, registered by Løvens Kemiske Fabriks Handelsaktiesel-skab, Denmark.

Devices of similar kinds but primarily intended for other body cavities are disclosed in the French Patent application with publication No 2 380 033 and in the U. S. Patent specification No 4 114 625. In connection with the prior art reference is also made to U.S. Patent specification No 4 178 934 relating to a urinary collecting device.

However, what is common to the previously known catheter devices intended for the inlet of air is either that they are relatively complicated and thereby expensive or that they require a rather large space or that complications arise sooner or later in connection with the utilized air filter. Among such complications there can be mentioned clogging of the filter and especially problems to keep the filter free from bacteria. In practice it has thus turned out to be extremely difficult to avoid the formation or inlet of bacteria at the aeration opening. This is a very difficult problem, to which it has hitherto not been possible to accomplish any satisfactory solution.

DESCRIPTION OF THE INVENTION:

By the catheter device according to the present invention is has shown possible to considerably reduce or eliminate the bacterium risk in connection with catheters, and this in spite of, or rather thanks to, the fact that it has been possible to make the device constructionally much simpler and thereby cheaper than previously known devices within this field. The new effect according to the invention is obtained by working with a gas channel which is totally closed off from the surrounding, and preferably also with a completely closed liquid channel and liquid collecting system as to the rest. The gas intended for entering into the liquid channel is thus collected from the gas which is present in the device already from the beginning, from the gas that is originally present in the liquid and gas channels as well as from the liquid collecting or storage means. In addition to the above-mentioned advantage from a bacterial point of view this also means a gain from a capacity point of view, as compared to previously known devices, where the liquid collecting container is filled not only with the intended liquid but also with gas that has been sucked in. Other known means intended to increase the capacity of the liquid collecting means, for instance by venting means therefrom, are complicated and also involve risks of having contaminations and infections spread via vented gas.

The catheter device according to the invention is thus characterized in that the gas channel comprises two openings, the first opening of which ends in the liquid storage means and the second opening of which ends in the liquid channel upstreams the outlet of the liquid channel in the liquid collecting means, and that the remainder of the gas channel is closed from the surroundings, whereby a closed gas circuit is obtained, wherein the gas that is intended to be let into the liquid channel is taken from the liquid storage or collecting means.

In this connection, for the continued description of the invention it should be noted that expressions such as "upstreams" and "downstreams" are related to the flow of liquid from the body cavity to the liquid storage or collecting means. This in turn means that each point of the catheter device is always considered to be downstreams the body cavity but upstreams the liquid storage means, independent of how the point referred to is vertically arranged relative to the body cavity or to the liquid storage means, respectively.

In this connection, it could also be added that with reference to the term "gas" the most natural gas to use is of course air, which is readily available. In order to reduce the growth of possibly occurring microorganisms in the closed system, however, other gases than air, for instance nitrogen or carbon dioxide, can be used, whereby especially the growth of aerobically functioning microorganisms is retarded.

As is clear from what has been mentioned above the primary object of the invention is that the gas channel is closed so as not to take any gas from the environment via any kind of a bacterium filter. Without deviating from the spirit of the invention, however, it is possible to introduce a filter in the gas channel if it is judged suitable to clean the circulating gas in the closed system.

Preferably also the liquid collecting means and the remainder of the total drainage means are shielded from the environment such that a completely closed system is obtained. However, the new closed gas channel implies already in itself a considerable improvement of the prior art in this field, which means that the device according to the invention is useful also as an improvement of known devices where gas can be allowed to enter the liquid channel or the liquid collecting means, that is at any other place than in the gas channel per se. The big advantage in connection with the invention is, however, reached when the system is completely closed. According to one embodiment of the catheter device according to the invention the gas channel is provided with a valve which permits flow of fluid in the direction from the liquid collecting means only. In this way liquid is prevented from flowing from the body cavity down into the gas channel. This valve, which can thus be termed a check valve, can for instance be a float valve. Thus, such a valve also works in such a way that re-suction of liquid into the gas channel is prevented when utilizing the closed system according to the invention.

Especially when using the catheter device for the drainage of a urinary bladder the design can be made also in other ways, for instance with a controlled valve, which is opened at a certain contraction pressure in the bladder and is then kept open for a certain time or to a certain level of pressure in the bladder. Such a valve can for instance be controlled electronically or with delay in any other way, whereby it will be possible to avoid the suspension of the catheter device in a bend, which will be described more below and which is otherwise most suitable for bedridden patients.

According to another preferable embodiment of the device according to the invention it has, however, turned out possible to make the gas channel completely free from fluid controlling valves. By this, a simple, inexpensive and reliable construction is achieved.

According to the invention it has also shown preferable to design the gas channel narrower than the liquid channel in that portion which is present in the catheter device as well as in the catheter which is connected to said device. In other words, in such a case the average cross section area of the gas channel shall be less than that of the liquid channel. The purpose according to the invention can then be achieved as soon as the cross section area of the gas channel is smaller than that of the liquid channel, but according to an especially preferable embodiment of the device according to the invention a considerably smaller cross section area is imparted to the gas channel than to the liquid channel. This can for instance mean that the average ratio between the cross section area of the gas channel and the cross section area of the liquid channel is less than about 1/4 and preferably less than about 1/10. The favourable effect in such a case with a more narrow gas channel is probably primarily due to the fact that the capillary forces are greater in the narrow gas channel than in the liquid channel. The invention is, however, not limited to any special theory of said effect.

According to still another preferable embodiment of the catheter device according to the invention it is shaped or produced in a way which at the time of use permits the arrangement of a vertical bend, the highest point of which is at a higher level than that end of the catheter device, or the catheter, which is intended to be inserted into the body cavity, and that the second opening of the gas channel is situated between said body cavity end and the first point upstreams the liquid collecting means which is at a higher level than that of the body cavity end. This means that suitably the drainage means is flexible, for instance a flexible tube, so as to enable some part thereof to be hung up at a point which is vertically higher situated than the end of the catheter or catheter device entered into the body cavity. Another alternative is of course to manufacture the device in a certain material, for instance a plastic, and then to impart to the same the desired shape, for suspension, already from the beginning.

The above-mentioned dimensioning of the liquid channel and gas channel, i.e. with a considerably narrower gas channel than liquid channel, is especially advantageous for the embodiment with a vertical bend mentioned in the previous paragraph. However, in this connection it can be mentioned that an effect which fundamentally corresponds to the effect obtained by the proper dimensioning, should be achieved also by using some sort of a throttling means in the gas channel.

It is true that the above described arrangement of a vertical bend is previously known per se, but anyhow it means great advantages in connection with the new device according to the invention, preferably when the second opening of the gas channel for the inlet of gas into the liquid channel is situated as close to the previously mentioned body cavity end as is possible and suitable for practical reasons.

However, independent of whether the catheter is used in a more or less straight shape or arranged with a bend, an especially preferable embodiment of the invention means that the second opening of the gas channel for the entrance of gas into the liquid channel is situated as close to the body cavity end as possible, that is preferably on essentially the same height or vertical level as the inlet opening of the liquid channel. In this way, it is essentially completely avoided that liquid will remain in the catheter device, at least in the case when the catheter device works as a catheter per se. On the other hand, if the device according to the invention is intended to be connected to a conventional catheter, it is true that liquid may remain in said catheter, but this usually means a relatively low negative pressure in the bladder, which does not cause any trouble to speak of. By such a placing of the second opening of the gas channel liquid is eliminated from the main part of the drainage means between body cavity and liquid collecting container.

If it is desired to avoid that gas be sucked in from the gas channel to the liquid channel before the body cavity has been completely emptied, the gas channel is preferably provided with a further opening that is arranged so as to be in direct contact with the body cavity when the catheter device is used. In this case, the most suitable and simple construction is to arrange said opening on mainly the same level as the inlet or upstream opening of the liquid channel in the device. The embodiment with the extra opening is primarily intended for those cases when the device is to be used per se as a catheter. In other cases there will be required a somewhat more sofisticated embodiment for the direct contact with the body cavity.

The natural way of accomplishing an opening from gas channel to liquid channel is of course to make a hole in the partition wall or walls between the two channels. However, according to the invention it has been shown that the functioning of the device is improved for instance if the opening of the gas channel into the liquid channel ends with a channel tip which extends in the downstream direction of the liquid channel. By the fact that the opening protrudes into a tip which is bended downstreams in the liquid channel, the risk will be reduced for a clogging of the gas channel by coagel or similar semisolid or solid substances possible occurring in the urine. Furthermore, through the fact that the cross section area of the urine channel at the tip opening of the gas channel will be smaller than in the rest of the urine channel, a venturi effect is achieved, which has a favourable effect on the resuction of the gas from the liquid collecting means.

Another constructionally simple embodiment of the opening of the gas channel into the liquid channel, which embodiment relates to a case where the catheter device is intended to be connected to an already existing catheter, implies that in its upstream end the device ends in a separate gas channel opening and a separate liquid channel opening, without any hole in the partition wall therebetween. In this way the opening from the gas channel into the liquid channel is formed via the outlet or downstream opening of the catheter or via an adapter device.

Due to the surface activity properties or urine foam can be formed when the gas leaves the gas channel, which may mean that the siphon effect will not be broken. This problem can be overcome by arranging a buffer volume of gas between the gas channel outlet and the gas inlet of the liquid channel, where the liquid channel inlet has a larger area than that of the outlet of the gas channel into the buffer voluma.

If the catheter device is to be connected to an already existing catheter which in itself contains an aeration channel, for instance a catheter for sump drain, the gas and liquid channels of the device are of course connected to the respective channel of the catheter.

As regards the arrangement of the drainage means in the liquid collecting means, a preferable embodiment means that the first opening of the gas channel into the liquid collecting means ends or opens at a vertically higher level than and outside the opening of the liquid channel in the liquid collecting means. In other words, the opening of the gas channel is at a higher level than the opening of the liquid channel, the risk of having liquid recycled with gas from the liquid collecting means being reduced. By such a construction with the opening of the gas channel at a higher level than that of the liquid channel in the liquid collecting means stagnant liquid in the gas channel is also avoided.

In order to further reduce the risk of recycling urine from the liquid collecting means to the gas channel, or, which is perhaps even more important, the risk of recycling microorganisms which may have been formed in the liquid collecting means, the liquid collecting means can be provided with a pre-chamber which is fitted with a check valve means of any known type or which extends down to the vicinity of the bottom of the liquid collecting means so as to form a liquid trap. According to another embodiment the pre-chamber can alternatively be arranged in the drain means and outside the liquid collecting means.

Due to the fact that the device according to the invention is of a simple construction and can thus be produced cheaply, it can be manufactured with liquid collecting means and drainage means in an integral part, for instance in the form of a plastics bag with attached plastic or rubber tubes. When the bag is filled with liquid, the whole bag with its connecting tubes can then be replaced by a new device without any substantial risk of having a contamination by microorganisms from the outside. However, the liquid collecting means need not be manufactured integrally with the tubes but can be used as a separate unit, which is changed in any conventional way, when it has been filled up, while taking into consideration the usual precautionary measures to keep the system sterile. According to a preferable embodiment the above-mentioned pre-chamber can then be positioned within the drain means. The liquid collecting means can of course also be emptied in a manner known per se by any device arranged in the bottom thereof.

DRAWINGS:

The invention will now be described more in detail in connection with the accompanying drawings, in which:

Figure 1 shows a side view in cross section of an embodiment of the device where it functions as a catheter per se;

Figure 2 shows a side view in cross section of another embodiment of the device according to the invention, where the device is intended to be connected to a catheter known per se;

Figure 3a shows an embodiment, in cross section, of the second opening of the gas channel into the liquid channel; and

Figure 3b shows another embodiment, in cross section, of the second opening of the gas channel into the liquid channel; and

Figure 3c shows still another embodiment, in cross section, of the second opening of the gas channel into the liquid channel, via a buffer volume; and

Figure 4 shows an embodiment, in cross section, where the device includes a pre-chamber arranged outside the liquid collecting means.

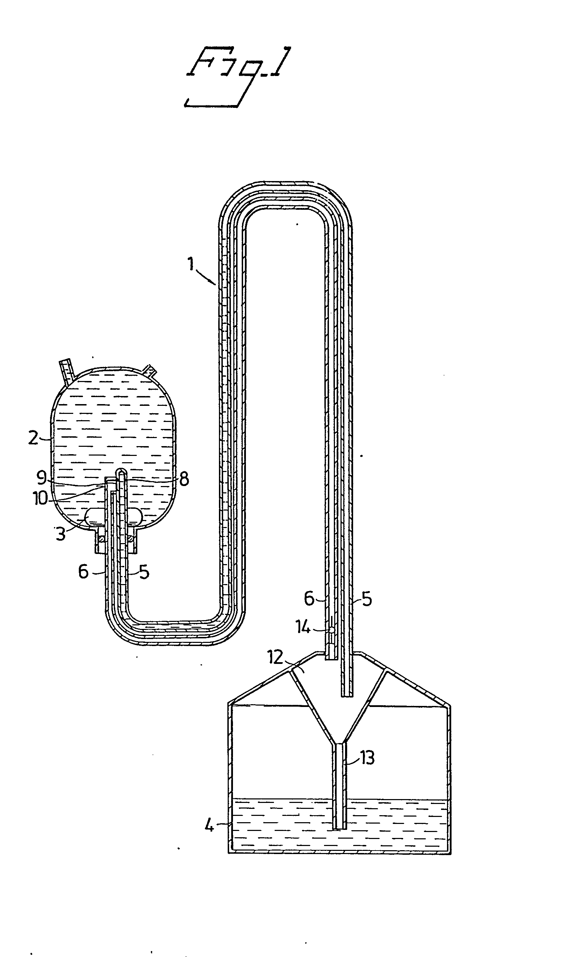

Thus, in figure 1 there is shown an embodiment, where the device according to the invention is intended to work as a catheter per se and where the device is provided with a vertically arranged bend.

The catheter, which is in the figure generally referred to as 1, is inserted with one end thereof in the urinary bladder 2 of a patient, where it is kept in place by a balloon 3. The other end of the catheter opens into a urine bag 4. Between its two ends the catheter is suspended in a bend with the top thereof at a level which is higher than that end of the catheter which is inserted into the urinary bladder 2. The catheter is of the three-channel type with one channel 5 for the drainage of urine, one channel 6 for the inlet of gas into the urine channel 5 and one (not shown) channel for the inflation of the balloon 3.

Inside the bladder 2 the urine channel 5 is provided with an opening 8 for inlet of urine from the bladder into the channel. In the figure the opening of the gas channel 6 into the liquid channel 5 has been designated reference numeral 9, and said opening 9 is, as is shown, arranged at the same level as the opening 8 in the urine channel. The gas channel 6 is further provided with an opening 10, which is in direct connection with the bladder 2. Also last-mentioned opening 10 is situated on the same level as the opening 8 adjacent to the urine channel 5, that is the openings 8, 9, and 10 are at essentially the same level.

In the shown case the urine bag 4 is provided with a pre-chamber 12, which narrows downwards and ends in a more narrow channel 13, which opens adjacent to the bottom of the bag. In the gas channel 6, which besides open at a higher level in the urine bag 4 than the urine channel 5, there is also a check valve 14, which is designed so as to permit flow of gas or liquid merely in the direction towards the urinary bladder.

The shown device works in the following way. When the pressure in the bladder 2 exceeds the counter pressure from the difference in height caused by the bend of the catheter tube, the urine flows out through the channel 5 into the urine bag 4, and the bladder is emptied by the siphon thus formed. When the bladder has been emptied, there will be a negative pressure therein, and gas will now be sucked in from the urine bag 4, that is from the gas volume above the liquid therein, through the channel 6 and via the opening 9 into the urine channel 5 and break the siphon action therein. In this connection, it should be added that merely a small amount of gas is needed to break such a siphon action.

The urine is conveyed through the hole 8 into the urine channel 5. In order to avoid that gas be sucked in through the opening 9 into the urine channel 5 before the urinary bladder has been completely emptied, the extra opening 10 is arranged, which opening is connected to the bladder and secures that the bladder is drained independent of the dimensioning of the gas and liquid channels. In certain cases, for instance in short time uses of the catheter, the bladder need not be completely emptied, and in such a case the opening 10 can be dispensed with. In other designs of the gas channel 6, or rather of the opening 9, which will be described more below, the opening 10 can also be omitted.

The function of the pre-chamber 12 and its channel 13 and of the valve 14 in the gas channel 6 has been described above and need not be repeated here.

The embodiment shown in figure 1 relates to a case where the gas channel 6 is of a dimension which does not significantly differ from the dimension of the urine channel 5. In such a case a check valve 14 is preferably used. If, however, the gas channel 6 is dimensioned to be significantly narrower than the urine channel 5, the check valve 14 can be omitted. In such a case, for the embodiment according to figure 1, liquid, that is urine, will first rise in the two channels, i.e. in the left leg of the U which is turned upside down in the figure. It has turned out that the liquid in the urine channel 5 will firstly pass the bend and give rise to a siphon effect therein. The flow pattern will then give rise to a negative pressure in the opening 9, whereby the urine that was present in the gas channel 6 will be resucked, whereupon gas from the bag 4 will empty the urine channel 5 of liquid.

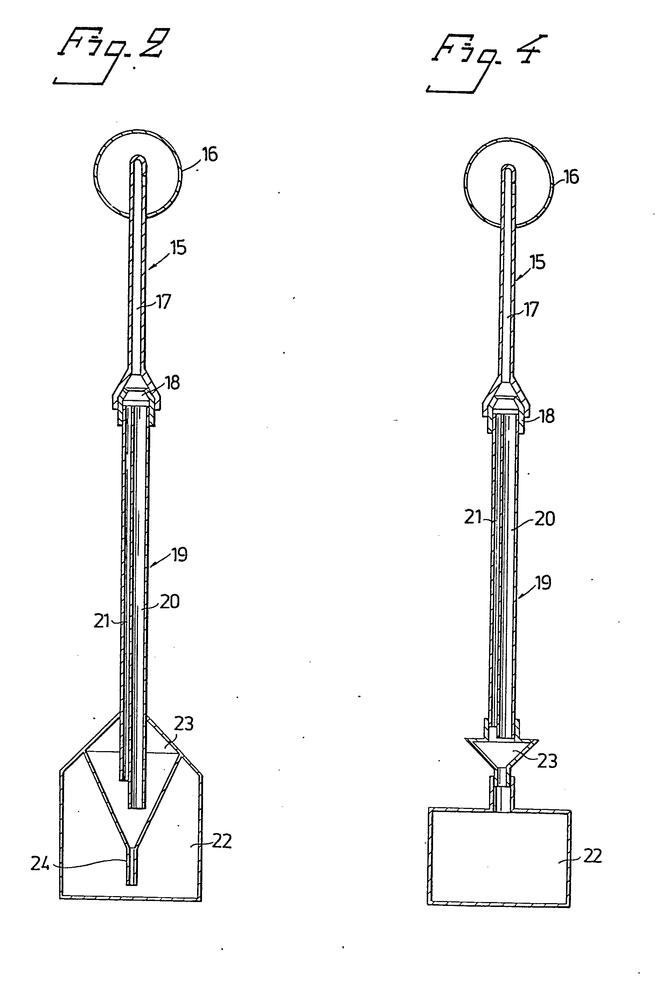

The embodiment shown in figure 1 and with a bend in the catheter tube possesses the advantage in connection with the drainage of a urinary bladder that it enables an intermittent drainage thereof. However, if the catheter tube is not suspended into a bend, the intermittent function will not appear, but still the formation of a continuous column of liquid in the urine tube will be prevented, and contrary to the prior art without letting gas or air into the system from the outside. Such an embodiment is shown in figure 2, where the device according to the invention partly comprises a straight construction, partly does not work as a catheter per se but is intended to be utilized as a catheter attachment.

Thus, in figure 2 there is shown a conventional catheter 15 which has been inserted into a urinary bladder 16 and which contains a urine channel 17. The urine channel 17 ends at the downstream end of the catheter 15, in a tube connection part 18. To said part 18 a catheter device 19 according to the invention is connected, which device comprises a urine channel 20 and a considerably more narrow air channel 21. The urine channel 20 as well as the air channel 21 open at the downstream end of the device 19 into a urine bag 22. This bag 22 has been designed in the same way as the bag shown in figure 1, that is with a pre-chamber 23 and a liquid lock 24. As is shown

0041487

18

in the figure the downstream end of the catheter device 19, that is the urine channel 20 as well as the air channel 21, open into the pre-chamber 23 of the bag 22. In its upstream end the device 19 is connected to the part 18 which is adapted to the catheter 15. The opening from the air channel 21 into the urine channel 20 can thus be considered to be the connection part 18. From the figure it is also clear that in this case there is not any check valve present in the air channel 21.

The function of the catheter device shown in figure 2 is principally the same as that of the device shown in figure 1. When the bladder 16 has been emptied of urine, a negative pressure will thus appear therein, which causes air to be sucked from the pre-chamber 23 into the urine bag 22 up through the air channel 21 to the part 18, where the sucking action of the liquid column is broken. By this, there will not be any significant amount of urine remaining in the catheter device 19. It is true that a minor portion of urine will remain in the urine channel 17 of the catheter 15, but as was mentioned above the negative pressure caused thereby will be relatively small, which means that it does not cause any significant trouble. At all events, the use of the device according to the invention implies an essential improvement of the prior art also in that case.

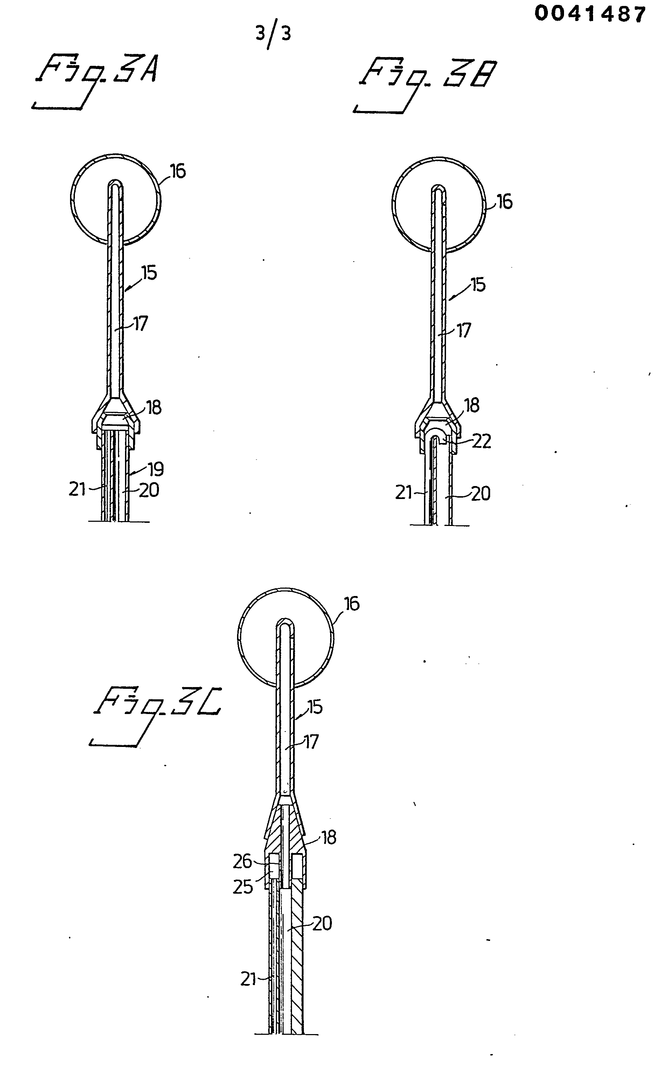

I figures 3a, 3b, and 3c there are shown three different embodiments of the second opening of the gas channel into the liquid channel. As in this case the catheter device according to the invention is utilized in the same way as in figure 2, i.e. connected to a conventional catheter, for the sake of simplicity the same reference numerals are used for the same details which appear in figure 2. Therefore, the function of said details need not be repeated.

Figure 3a shows in a more detailed way the same embodiment of the opening of the gas channel 21 into the
liquid channel 20 as in figure 2. There is not any
opening in the partition wall between the liquid channel
20 and the gas channel 21, but the gas from the gas
channel 21 flows over into the liquid channel 20 via the
connecting part 18.

Figure 3b represents the embodiment wherein the second opening of the gas channel 21 into the liquid channel
20 ends with a curved tip 22. This flow-improving tip 22
is, as is shown, curved so as to extend in the downstream
direction of the liquid channel 20. This means is flow
promoting for the gas inlet by the fact that a venturi
effect appears at the opening of the gas channel into
the liquid channel.

Figure 3c shows more in detail the opening of the
gas channel 21 into the liquid channel 20 via a buffer
volume 25 within the adapter part 18. As was mentioned
above the opening 26 from the buffer volume 25 into the
liquid channel 20 is of a greater area than that of the
outlet from the gas channel 21 into said buffer volume
25.

Figure 4 represents the embodiment where the liquid
collecting means 22 is connected to the drain means via
a pre-chamber 23 arranged outside the liquid collecting
means. The reference numerals are the same as in figure
2.

Although the invention has been described in connection with the embodiments shown in the drawings, it is
of course not limited thereto, but modifications are
possible within the scope of the accompanying claims.
Thus, for instance the drain means may comprise concentrically arranged gas and liquid channels, e.g. with the
gas channel arranged within or inside the liquid channel,
etc.

CLAIMS:

1. Catheter device intended to be inserted into (1), or connected to an already existing catheter inserted into, a body cavity for draining liquid therefrom, e.g. for the drainage of fluid from a urinary bladder (2, 16), comprising a drain means, preferably a tube or a pipe, with at least one channel (5, 20) for said liquid and at least one channel (6, 21) for the inlet of gas, e.g. air, into the liquid channel (5, 20), and a liquid collecting means connected to said drain means, preferably a bag (4, 22), characterized in that the gas channel (6, 21) comprises two openings, a first one of which opens into the liquid collecting means (4, 22) and a second one of which (9, 22) opens into the liquid channel (5, 20) upstream the outlet from the liquid channel into the liquid collecting means (4, 22), and in that the rest of the gas channel (6, 21) is closed from the surrounding, whereby a closed gas circuit is obtained, wherein the gas intended for entrance into the liquid channel (5, 20) is taken from the liquid collecting means (4, 22).

2. Catheter device according to claim 1, characterized in that also the liquid collecting means (4, 22) and the rest of the drain means are closed from the surrounding, such that a completely closed system is obtained.

3. Catheter device according to claim 1 or 2, characterized in that the average sectional area of the gas channel (6, 21) is smaller than that of the liquid channel (5, 20).

4. Catheter device according to claim 3, characterized in that the average ratio between the sectional area of the gas channel (6, 21) and the sectional area of the liquid channel (5, 17, 20) is less than about 1/4, preferably less than about 1/10.

5. Catheter device according to any one of the preceding claims, characterized in that the gas channel (6) is provided with a valve (14) which permits flow of fluid in the direction from the liquid collecting means (4) only.

6. Catheter device according to any one of claims 1-4, characterized in that the gas channel (21) is free from fluid controlling valves.

7. Catheter device according to any one of the preceding claims, characterized in that it is shaped or manufactured in a way which at the time of use permits the arrangement of a vertical bend, the highest point of which is at a higher level than that end of the catheter device (1), or the catheter, which is intended to be inserted into the body cavity (2), and that the second opening of the gas channel (6) is positioned between said body cavity end and the first point which upstreams the liquid collecting means (4) is at a higher level than that of the body cavity end.

8. Catheter device according to any one of the preceding claims, characterized in that the second opening (9, 22) of the gas channel (6, 21) is positioned at essentially the same level as the inlet opening of the liquid channel (5, 20).

9. Catheter device according to any one of the preceding claims, characterized in that the gas channel (6) is provided with a further opening (10) which is arranged so as to be, at the time of use, in direct contact with the body cavity (2), preferably at essentially the same level as the inlet opening (8) of the liquid channel (5).

10. Catheter device according to any one of the preceding claims, which is intended to be connected to an already existing catheter, characterized in that it ends, in its upstream end, in a separate gas channel opening and a separate liquid channel opening so as to form said second opening from the gas channel (21) into the liquid channel (20) via the outlet opening of the catheter or an adapter part (18) connected thereto.

11. Catheter device according to claim 10, characterized in that the adapter part (18) contains a cavity (25) which connects the second opening of the gas channel (21) to the liquid channel (20) via an opening (26), the sectional area of which is preferably larger than that of said second opening of the gas channel.

12. Catheter device according to any one of the preceding claims, characterized in that the second opening of the gas channel (21) into the liquid channel (20) ends with a channel tip (22) which extends in the downstream direction of the liquid channel.

13. Catheter device according to any one of the preceding claims, characterized in that the first opening of the gas channel (6, 21) into the liquid collecting means (4, 22) opens at a vertically higher position than and outside the opening of the liquid channel into the liquid collecting means.

14. Catheter device according to any one of the preceding claims, characterized in that the liquid collecting means (4, 22) comprises a pre-chamber (12, 23) with a recycle-preventing means, for instance a check valve or a liquid trap (13, 24).

15. Catheter device according to any one of the preceding claims ,characterized in that the drain means is connected to the liquid collecting means (22) via a pre-chamber (23) outside said liquid collecting means, whereby the first opening of the gas channel (21) as well as the downstream opening of the liquid channel (20) open into said pre-chamber, said first opening preferably being at a vertically higher level than and outside that of the downstream opening of the liquid channel.

Fig.1

Fig. 2

Fig. 4

0041487

Fig.3A

Fig.3B

Fig.3C

16
15
17
18
19
21 20

16
15
17
18
22
21 20

16
15
17
18
26
25
20
21

# EUROPEAN SEARCH REPORT

European Patent Office

Application number

EP 81 85 0091.0

## DOCUMENTS CONSIDERED TO BE RELEVANT

| Category | Citation of document with indication, where appropriate, of relevant passages | Relevant to claim |
|---|---|---|
| X | <u>FR - A - 2 187 362</u> (KENDALL CO.)<br>* claims 1, 2, 3, 5, 7, 8, 9, 11 *<br>  &  US - A - 3 851 650<br>  &  DE - A - 2 328 613<br>-- | 1,2,5,<br>6,13,<br>14,15 |
| | <u>US - A - 3 583 401</u> (VAILLANCOURT et al.)<br>* complete document *<br>-- | 1,6,8,<br>14 |
| | <u>GB - A - 1 502 734</u> (JARUND DEVELLO<br>  AB)<br>* claim 1; column 2, lines 78 to 104 *<br>-- | 7 |
| A | <u>FR - A1 - 2 360 319</u> (KENDALL CO.)<br>* claims 1, 8; page 1, lines 12<br>  to 19 and 32 to 37; page 6, lines<br>  33 to 36 *<br>-- | |
| A | <u>US - A- 3 672 372</u> (H.J. HEIMLICH)<br>* abstract *<br>---- | |

### CLASSIFICATION OF THE APPLICATION (Int. Cl.³)

A 61 M   25/00
A 61 M   1/00
A 61 F   5/44

### TECHNICAL FIELDS SEARCHED (Int. Cl.³)

A 61 F   5/44
A 61 M   1/00
A 61 M   25/00

### CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

The present search report has been drawn up for all claims

| Place of search | Date of completion of the search | Examiner |
|---|---|---|
| Berlin | 20-08-1981 | CLOT |

EPO Form 1503.1   06.78